# FASCICULE DE BREVET EUROPEEN

(11) **EP 2 039 343 B1**
(45) Date de publication et mention de la délivrance du brevet: **05.12.2012**
(21) Numéro de dépôt: 08164230.8
(22) Date de dépôt: 12.09.2008
(51) Int. Cl.: A61K 8/81, A61K 8/898, A61Q 5/02, A61Q 5/12

(54) **Compositions cosmétiques contenant un copolymère cationique et une silicone aminée particulière et leurs utilisations.**
Kosmetische Zusammensetzungen die ein kationisches Copolymer und ein bestimmtes Aminosilikon enthalten, und deren Verwendung
Cosmetic compositions containing a cationic copolymer and a particular amino silicone and uses thereof

(30) Priorité: 14.09.2007 FR 0757566
(43) Date de publication de la demande: 25.03.2009
(73) Titulaire: L'Oréal, 75008 Paris (FR)
(72) Inventeur: Biganska, Olga, 92600 Asnières sur Seine (FR); Chesneau, Laurent, 92300 LEVALLOIS PERRET (FR)
(74) Mandataire: Le Blainvaux Bellegarde, Françoise

(56) Documents cités:
- WO-A-2005/092276
- FR-A- 2 798 852
- FR-A- 2 831 800
- ALLEN PARK ET AL.: "Carbopol Aqua CC Polymer: The Premier Cationic Compatible Rheology modifier for Low pH Formulations" COSMETIC SCIENECE TECHNOLOGY, [Online] 14 novembre 2006 (2006-11-14), page 241, XP002476985 Extrait de l'Internet: URL:http://web.archive.org/web/20061114121 411/http://www.personalcare.noveon.com/Pre sentationsAndPublications/noveon2.pdf> [extrait le 2008-04-16]
- ANONYMOUS: "Deep Conditioner for Ethnic Hair E-0018"[Online] 13 septembre 2007 (2007-09-13), XP002476803 Extrait de l'Internet: URL:http://www.personalcare.noveon.com/for mulas/E-0018.pdf> [extrait le 2008-04-16]
- ANONYMOUS: "Hair conditioning mask CD-0016"[Online] 30 octobre 2007 (2007-10-30), XP002477360 Extrait de l'Internet: URL:http://www.personalcare.noveon.com/for mulas/CD-0016(AP).pdf> [extrait le 2008-04-21]
- ANONYMOUS: "SilSense?* A-21 & A-23 Silicones Amino-Functional Silicones"[Online] 27 juin 2005 (2005-06-27), XP002476804 Extrait de l'Internet: URL:http://www.personalcare.noveon.com/Tec hnicalDataSheets/TDS-321_SilSense_A-21_A-2 3.pdf> [extrait le 2008-04-16]

## Description

La présente invention concerne de nouvelles compositions cosmétiques comprenant dans un milieu cosmétiquement acceptable une ou plusieurs silicones aminées particulières et un ou plusieurs copolymères cationiques particuliers.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou amphotères ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).
En outre, l'usage des polymères cationiques ou amphotères dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

Les silicones aminées sont généralement utilisées dans les compositions de shampooings en tant qu'agents conditionneurs pour améliorer la douceur, le toucher et le démêlage des cheveux. Cependant, on a constaté que ces silicones conduisaient à la formation d'une couche inesthétique à la surface du shampooing nuisible aux performances du shampooing. Pour éviter l'apparition de ce phénomène, des agents de stabilisation tels que les polymères acryliques réticulés du type Carbopol sont fréquemment utilisés. Néanmoins, ces agents de stabilisation présentent l'inconvénient de diminuer les performances cosmétiques des shampooings, notamment en rendant les cheveux plus chargés et plus rêches.

Il était donc nécessaire de mettre au point une composition cosmétique détergente, en particulier un shampooing, qui permette d'obtenir de bonnes performances cosmétiques sur les matières kératiniques, à savoir notamment les cheveux et le cuir chevelu, et plus particulièrement sur les cheveux naturels.

En résumé, il s'avère que les compositions cosmétiques actuelles contenant des silicones aminées, ne donnent pas encore complètement satisfaction.

On connaît dans l'état de la technique des compositions cosmétiques en particulier détergentes contenant un copolymère cationique comme agent de stabilisation ou de suspension d'ingrédients insolubles dans l'eau comme des silicones ou des corps gras. De telles compositions ont été décrites notamment dans la demande de brevet WO2005/092276. Les qualités de mousse et les propriétés cosmétiques obtenues avec ces compositions ne sont pas encore suffisamment satisfaisantes, de même que les propriétés cosmétiques.

La demanderesse a maintenant découvert que l'association d'un copolymère cationique particulier et d'une silicone aminée particulière permet de remédier à ces inconvénients.
En effet, il a été constaté que l'utilisation dudit copolymère cationique dans les compositions de la présente invention permettait d'obtenir sur les matières kératiniques, notamment les cheveux de très bonnes propriétés cosmétiques particulièrement au niveau de la légèreté, de la douceur, du lissage au toucher, au niveau de la souplesse et de la malléabilité sur cheveux séchés. Il a aussi été constaté que les compositions de l'invention permettaient d'obtenir des cheveux séchés présentant au regard un aspect général plus lisse.

Par ailleurs, les compositions selon l'invention présentent une bonne rhéologie, sont stables et présentent un aspect visuel esthétique.

De plus, dans le cadre des compositions moussantes en particulier des shampooings, les propriétés de la mousse (aspect, consistance, abondance de la mousse, élimination de la mousse) sont très satisfaisantes. En particulier, le démarrage de la mousse est rapide et la mousse s'élimine facilement.

Les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable,
(i)- un ou plusieurs polymères cationiques qui sont produits par polymérisation d'un mélange de monomères comprenant :
   a) un ou plusieurs monomères vinyliques substitués par un ou plusieurs groupes amino,
   b) un ou plusieurs monomères vinyliques non ioniques hydrophobes,
   choisis parmi ceux de formules (I) et (II) :

   (I) CH₂=C(X)Z,

   (II) CH₂=CH-OC(O)R;

   dans lesquelles formules (I) et (II) :
   X représente H ou un groupe méthyle ;
   Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
   x représente un nombre entier allant de 1 à 6 ;
      chaque R représente indépendamment un groupe alkyle en C₁-C₃₀ ;
      chaque R¹ représente indépendamment un groupe alkyle en C₁-C₃₀ , un groupe alkyle en C₂-C₃₀ hydroxylé, ou un groupe alkyle en C₁-C₃₀ halogéné et
   c) un ou plusieurs monomères vinyliques associatifs,
   e) un ou plusieurs monomères vinyliques non ioniques hydroxylés, et
(ii)- une ou plusieurs silicones aminées telles que définies ci-dessous.

L'invention a également pour objet l'utilisation d'une composition telle que définie ci-dessus pour apporter une ou plusieurs propriétés choisies parmi la brillance, la légèreté, la douceur, le lissage et la souplesse des cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.
Par monomère hydrophobe, on entend au sens de la présente invention, un monomère qui présente une solubilité dans l'eau inférieure à 10g pour 100 mL d'eau à une température de 20°C.

Un autre objet de l'invention concerne l'utilisation d'un copolymère cationique (i) tel que décrit ci-dessus dans, ou pour la fabrication d'une composition cosmétique comprenant une ou plusieurs silicones aminées telles que définies ci-dessous.

Les différents objets de l'invention vont maintenant être détaillés. L'ensemble des significations et définitions des composés utilisés dans la présente invention données ci-dessous sont valables pour l'ensemble des objets de l'invention.

L'une des caractéristiques essentielles de l'invention est la présence d'un polymère cationique qui est obtenu par polymérisation d'un mélange de monomères comprenant a) un ou plusieurs monomères vinyliques substitués par un ou plusieurs groupes amines, b) un ou plusieurs monomères vinyliques non ioniques hydrophobes, c) un ou plusieurs monomères vinyliques associatifs et e) un ou plusieurs monomères vinyliques non ioniques hydroxylés. De préférence, les monomères constituant le copolymère cationique comprennent en outre un ou plusieurs monomères tensioactifs vinyliques semi-hydrophobes d). Les monomères a) à e) sont différents les uns des autres.

De préférence, le polymère cationique (i) est un polymère épaississant.

Au sens de la présente invention, on entend par polymère épaississant, un polymère qui, introduit à 1 % en poids dans une solution aqueuse ou hydroalcoolique à 30 % en poids d'éthanol, et à pH 7, permet d'atteindre une viscosité d'au moins 100 cps à 25 °C, et à un taux de cisaillement de 1 s⁻¹. Cette viscosité peut être mesurée à l'aide d'un viscosimètre à géométrie cône-plan, par exemple, un rhéomètre Haake RS 600. De préférence, ces polymères permettent d'augmenter la viscosité des compositions dans lesquelles ils se trouvent, d'au moins 50 cps à 25 °C et à 1 s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant un ou plusieurs groupes R₀CH=C(R₀)- , ou chaque R₀ est indépendamment H, un alkyle en C₁-C₃₀, -C(O)OH ou C(O)OR₀', -OC(O)OR₀', -C(O)NHR₀', C(O)NRo' R₀" ,R₀' et Ro", identiques ou différents, étant un groupe alkyle en C₁-C₃₀.
Ainsi, par exemple, au sens de la présente invention, les (méth)acrylates et les (méth)acrylamides sont des monomères vinyliques.
Comme expliqué précédemment, le mélange de monomères permettant la préparation du polymère cationique (i) utilisé dans la composition selon l'invention comprend un ou plusieurs monomères vinyliques substitués par un ou plusieurs groupes amino.
Le ou les monomères vinyliques substitués par un ou plusieurs groupes amines sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides à groupement(s) hétérocyclique(s) contenant un atome d'azote,
- les (méth)acrylates à groupement(s) hétérocyclique(s) contenant un atome d'azote,
- les hétérocycles azotés à groupement(s) vinyle(s), et leurs mélanges.
   A titre de monomères vinyliques substitués par un ou plusieurs groupes amino préférés, on peut citer :

- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.
   Lorsque les monomères sont sous forme de sels, il peut s'agir de sels minéraux, tels que les sels hydrochlorure, sulfate et phosphate ; ou bien de sels d'acides organiques, tels que les sels acétate, maléate et fumarate.

Des monomères vinyliques substitués par un ou plusieurs groupe(s) amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.
   Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.
   Comme expliqué précédemment, le mélange de monomères permettant la préparation du polymère cationique utilisé (i) selon l'invention comprend également un ou plusieurs monomères vinyliques non ioniques hydrophobes b).
   Le ou les monomères vinyliques non ioniques hydrophobes utilisables pour la préparation du polymère cationique utilisé selon l'invention sont généralement choisis parmi les composés de formules (I) et (II) :

   (I) CH₂=C(X)Z,

   (II) CH₂=CH-OC(O)R;
où, dans chacune des formules (I) et (II) ;
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
   chaque R représente indépendamment un groupe alkyle en C₁-C₃₀ ;
   chaque R¹ représente indépendamment un groupe alkyle en C₁-C₃₀ , un groupe alkyle en C₂-C₃₀ hydroxylé, ou un groupe alkyle en C₁-C₃₀ halogéné.
   On peut citer en particulier les (méth)acrylates d'alkyle en C₁-C₃₀ ; les (alkyl en C₁-C₃₀)(méth)acrylamides ; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile ; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.
   De préférence, le ou les monomères vinyliques non ioniques hydrophobes sont choisis parmi les acrylates d'alkyle en C₁-C₃₀, les méthacrylates d'alkyle en C₁-C₃₀, et leurs mélanges, tels que l'acrylate d'éthyle, le méthacrylate de méthyle, le méthacrylate de 3,3,5-triméthylcyclohexyle.
   Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé selon l'invention sont généralement choisis parmi des composés ayant une extrémité (i)' à insaturation(s) éthylénique(s) pour la polymérisation par addition avec d'autres monomères du système ; une portion centrale (ii)' polyoxyalkylène pour conférer des propriétés hydrophiles sélectives aux polymères, et une extrémité (iii)' hydrophobe pour conférer des propriétés hydrophobes sélectives aux polymères.
L'extrémité (i)' à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i)' du monomère associatif peut être dérivée d'un allyl éther ou d'un vinyl éther ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.
La portion centrale (ii)' du ou des monomères vinyliques associatifs est de préférence un segment polyoxyalkylène comprenant 5 à 250, de préférence encore 10 à 120, et mieux 15 à 60 unités oxydes d'alkylène en C₂-C₇. Des portions centrales (ii)' préférées sont les segments polyoxyéthylène, polyoxypropylène, et polyoxybutylène comprenant 5 à 150, de préférence 10 à 100, et mieux 15 à 60 unités oxydes d'éthylène, de propylène ou de butylène, et des séquences aléatoires ou non aléatoires d'unités oxydes d'éthylène, oxydes de propylène ou oxydes de butylène. De préférence, les portions centrales sont des segments polyoxyéthylène.
L'extrémité (iii)' hydrophobe du ou des monomères associatifs est de préférence un fragment hydrocarboné appartenant à l'une des classes hydrocarbonées suivantes : un alkyle linéaire en C₈-C₄₀, un alkyle en C₂-C₄₀ substitué par un groupe aryle, un phényle substitué par un groupe alkyle en C₂-C₄₀, un alkyle ramifié en C₈-C₄₀, un groupe alicyclique en C₈-C₄₀, et un ester complexe en C₈-C₈₀.
Par ester complexe, on entend au sens de la présente invention tout ester différent d'un ester simple.
Par ester simple, on entend au sens de la présente invention tout ester d'alcool aliphatique saturé en C₁-C₃₀, linéaire ou ramifié, et non substitué.

Des exemples d'extrémités (iii)' hydrophobes du ou des monomères associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).
Des exemples de groupes alkyles linéaires ou ramifiés ayant 8 à 40 atomes de carbone et dérivés d'une source naturelle sont notamment les groupes alkyles dérivés de l'huile d'arachide hydrogénée, d'huile de soja et d'huile de canola (à prédominance C₁₈), l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié), le phytol hydrogéné (C₂₀ ramifié).
Des exemples de phényles substitués par un alkyle en C₂-C₄₀ sont l'octylphényle, le nonylphényle, le décylphényle, le dodécylphényle, l'hexadécylphényle, l'octadécylphényle, l'isooctylphényle et le secbutylphényle.
Des groupes alicycliques en C₈-C₄₀ peuvent être par exemple les groupes dérivés des stérols d'origine animale, tels que le cholestérol, le lanostérol, le 7-déhydrocholestérol ; ou bien les dérivés d'origine végétales, tels que le phytostérol, le stigmastérol, le campestérol ; ou bien les dérivés issus de microorganismes, tels que l'ergostérol, le mycrostérol. D'autres alicycliques en C₈-C₄₀ utilisables dans la présente invention sont par exemple le cyclooctyle, le cyclododécyle, l'adamantyle, le décahydronaphthyle, et les groupes dérivés de composés alicycliques en C₈-C₄₀ naturels tels que le pinène, le rétinol hydrogéné, le camphre et l'alcool d'isobornyle.
Les groupes alkyles en C₂-C₄₀ substitués par un groupe aryle peuvent être par exemple le 2-phényléthyle, le 2,4-diphénybutyle, le 2,4,6-triphénylhexyle, le 4-phénylbutyle, le 2-méthyl-2-phényléthyle, le 2,4,6-tri(1'phényléthyl)phényle.
A titre d'esters complexes en C₈-C₄₀ utilisables comme extrémité (iii), on peut citer l'huile de ricin hydrogénée (principalement le triglycéride de l'acide 12-hydroxystéarique ; les 1,2-diacyl glycérols tels que le 1,2-distéaryl glycérol, le 1,2-dipalmityl glycérol, le 1,2-dimyristyl glycérol ; les di-, tri- ou poly-esters de sucres tel que le 3,4,6-tristéaryl glucose, le 2,3-dilauryle fructose ; et les esters de sorbitane tels que ceux divulgués dans le brevet US 4,600,761.
Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.
De préférence, le ou les monomères vinyliques associatifs c) utilisables selon l'invention sont choisis parmi les composés de formule (III) :

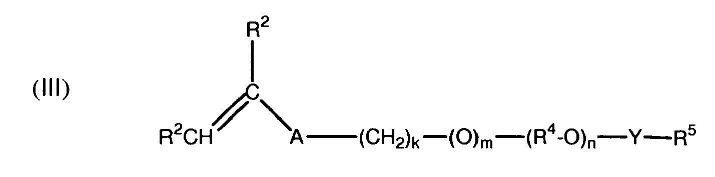

Dans laquelle
chaque R² est indépendamment H, un groupe méthyle, un groupe
- C(O)OH, ou un groupe -C(O)OR³ ;
   R³ est un alkyle en C₁-C₃₀ ;
   A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)-;
   Ar est un groupe aryle divalent ;
   E est H ou un groupe méthyle ;
   z est égal à 0 ou 1 ;
   k est un entier variant de 0 à 30 ;
   m est égal à 0 ou 1, à la condition que lorsque k = 0, m = 0, et lorsque k varie de 1 à 30, m est égal à 1 ;
   (R⁴-O)ₙ est un polyoxyalkylène, qui est un homopolymère, un copolymère aléatoire, ou un copolymère à blocs, avec des unités oxyalkylène en C₂-C₄,
   R⁴ est C₂H₄, C₃H₆, C₄H₈, ou leurs mélanges,
   n est un entier variant de 5 à 250,
   Y est -R⁴O-, -R⁴NH-, -C(O)-, -C(O)NH-, R⁴NHC(O)NH-, ou -
   C(O)NHC(O)-;
   R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
   le groupe alkyle R⁵ comprenant éventuellement un ou plusieurs substituants choisis parmi les groupes hydroxyle, alcoxyle et halogèno.
   De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.
   Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 unités oxydes d'éthylène.
   De préférence le ou les monomères associatifs vinyliques représentent de 0,001 à 25 % en poids, de préférence de 0,01 à 15 % en poids et mieux de 0,1 à 10 % en poids du mélange de monomères.

Le ou les monomères tensioactifs vinyliques semi-hydrophobes, éventuellement présents dans le mélange de monomères peuvent modérer les propriétés associatives des polymères associatifs cationiques qui les contiennent, produisant ainsi des gels aqueux ayant une très bonne texture et de très bonnes propriétés rhéologiques.
Par monomère tensioactif vinylique semi-hydrophobe, on entend au sens de la présente invention un monomère qui a une structure similaire à un monomère associatif, mais a une extrémité substantiellement non hydrophobe et ainsi ne confère pas de propriété associative aux polymères.
La propriété d'associativité d'un polymère est liée à la propriété dans un milieu donné, des molécules dudit polymère de s'associer entre elles, ou de s'associer à des molécules d'un co-agent, en général tensioactif, ce qui se traduit dans un certain domaine de concentration par un accroissement de la viscosité du milieu.
Le ou les monomères tensioactifs vinyliques semi-hydrophobes sont généralement des composés ayant deux parties :
(i) un groupe terminal insaturé pour permettre la polymérisation par addition avec les autres monomères du mélange de réaction, et
(ii) un groupe polyoxyalkylène pour atténuer les associations entre les groupes hydrophobes du polymère ou les groupes hydrophobes des autres matériaux éventuellement présents dans la composition comprenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité A peut dériver d'un éther allylique, d'un éther vinylique ou d'un uréthane insaturé non ionique.
L'extrémité A insaturée polymérisable peut aussi dériver d'un acide gras insaturé en C₈-C₃₀ comprenant un ou plusieurs groupe fonctionnel carboxy libre. Ce groupe en C₈-C₃₀ fait partie de l'extrémité insaturée A et est différent des groupes hydrophobes pendant des monomères associatifs, qui sont séparés de l'extrémité insaturée du monomère associatif par un groupe espaceur hydrophile.
La portion B polyoxyalkylène comprend un segment polyoxyalkylène à chaîne longue, qui est essentiellement similaire à la portion hydrophile des monomères associatifs. Des portions polyoxyalkylène B préférées incluent les unités en C₂-C₄ polyoxyéthylène, polyoxypropylène, et polyoxybutylène comprenant de 5 à 250, de préférence de 10 à 100 unités oxyalkylène. Lorsque le monomère tensioactif vinylique semi-hydrophobe comprend plus d'un type d'unité d'oxyalkylène, ces unités peuvent être disposés en séquence aléatoire, non aléatoire, ou à bloc.
De préférence, le ou les monomères tensioactifs vinyliques semi-hydrophobes sont choisis parmi les composés de formules (IV) ou (V) : 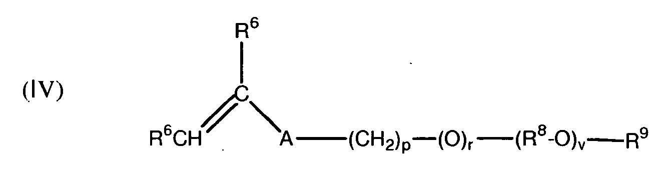 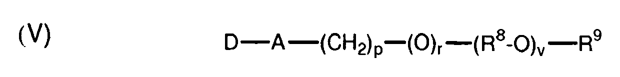 où, dans chaque formule (IV) ou (V),
chaque R⁶ représente indépendamment H, un alkyle en C₁-C₃₀, - C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH -C(O)NH-, -Ar-(CE₂)_{z}-NHC(O)O-, -Ar-(CE₂)_{z} -NHC(O)NH-, ou -CH₂CH₂NHC(O)-
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alkyle insaturé en C₈-C₃₀ ou un alkyle insaturé en C₈-C₃₀ substitué par un groupe carboxy.

De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H

ou

CH₂=CHCH₂O(C₃H₆O)_{d}(C₂H₄O)ₑH;

où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.
Des monomères tensioactifs vinyliques semi-hydrophobes préférés sont par exemple les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et
RAL 307.
Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O)₁₀H;
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O)₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O)₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂=CHCH₂-O(C₃H₆O)₅(C₂H₄O)₅H.
La quantité du ou des monomères tensioactifs vinyliques semi-hydrophobes utilisés dans la préparation des polymères cationiques (i) utilisés dans la composition selon l'invention peut varier largement et dépend, en autre, des propriétés rhéologiques finales désirées pour le polymère.
Lorsqu'ils sont présents, les monomères tensioactifs vinyliques semi-hydrophobes représentent de 0,01 à 25 % en poids et de préférence de 0,1 à 10% en poids par rapport au poids total du mélange de monomères.

Le ou les polymères cationiques (i) utilisés dans la composition selon l'invention sont préparés à partir d'un mélange de monomères pouvant comprendre un ou plusieurs monomères vinyliques non ioniques hydroxylés.
Ces monomères sont des monomères à insaturation(s) éthylénique(s) comprenant un ou plusieurs substituants hydroxyle, choisis parmi les (méth) acrylates d'hydroxyalkyle en C₁ - C₆, les (hydroxyalkyl en C₁ - C_{4 (méth) acrylamides, et leurs mélanges}
A titre de monomères vinyliques non ioniques hydroxylés, on peut citer les (méth)acrylates d'alkyle en C₁-C₄ hydroxylés, tel que le 2-hydroxyéthylméthacrylate (HEMA), le 2-hydroxyéthyl acrylate (2-HEA), le 3-hydroxypropyl acrylate ; le N-(2-hydroxyéthyl) méthacrylamide, le N-(2-hydroxyéthyl) acrylamide, le N-(3-hydroxypropyl) acrylamide, le N-(2,3-dihydroxypropyl) acrylamide ; et leurs mélanges. Lorsqu'il(s) est (sont) présent(s), le ou les monomères vinyliques non ioniques hydroxylés représentent généralement jusqu'à 10% en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques utilisés (i) dans la composition selon l'invention sont préparés à partir d'un mélange de monomères qui peut comprendre un ou plusieurs monomères réticulants permettant d'introduire des ramifications et de contrôler la masse moléculaire.
Des agents réticulants poly-insaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulant utilisables peuvent être des monomères polyfonctionnels comprenant des groupes réactifs multiples tels que des groupes époxydes, isocyanates et des groupes silanes hydrolysables. De nombreux composés poly-insaturés peuvent être utilisés pour générer un réseau tri-dimensionnel partiellement ou substantiellement réticulé.
Des exemples de monomères réticulants polyinsaturés utilisables sont par exemple les monomères aromatiques polyinsaturés, tel que le divinylbenzène, le divinyl naphtylène, et le trivinylbenzène ; les monomères alicycliques polyinsaturés, tel que le 1,2,4-trivinylcyclohexane ; les esters difonctionnels de l'acide phthalique, tel que le diallyl phthalate ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène, et le 1,5 heptadiène.
D'autres monomères réticulants polyinsaturés utilisables sont par exemple les éthers polyalcényles, tels que le triallyl pentaérythritol, le diallyl pentaérythritol, le diallyl saccharose, l'octaallyl saccharose et le triméthylolpropane diallyl éther ; les esters polyinsaturés de polyalcools ou de polyacides, tel que le 1,6-hexanediol di(méth)acrylate, le tétraméthylène tri(méth)acrylate, l'allyl acrylate, le diallyl itaconate, le diallyl fumarate, le diallyl maléate, le triméthylolpropane tri(méth)acrylate, le triméthylolpropane di(méth)acrylate et le polyéthylène glycol di(méth)acrylate, les alkylène bisacrylamides, tel que le méthylène bisacrylamide, le propylène bisacrylamide; les dérivés hydroxylés et carboxylés du méthylène bisacrylamide, tel que le N,N'-bisméthylol méthylène bisacrylamide ; les polyéthylèneglycol di(méth)acrylates, tels que l'éthylèneglycol di(méth)acrylate, le diiéthylèneglycol di(méth)acrylate, le triéthylèneglycol di(méth)acrylate, les silanes polyinsaturés tels que le diméthyldivinylsilane, le méthyltrivinylsilane, l'allyldiméthylvinylsilane, le diallyldiméthylsilane et le tétravinylsilane, les stannanes polyinsaturés, tel que le tétraallyl étain et le diallyldiméthyl étain.
Des monomères réticulants monoinsaturés utilisables et portant un groupe réactif peuvent être les N-méthylolacrylamides; les N-alkoxy(méth)acrylamides, où le groupe alkoxy est un groupe C₁-C₁₈ ; et les silanes hydrolysables insaturés tel que triéthoxyvinylsilane, le trisisopropoxyvinylsilane, et le 3-triéthoxysilylpropyl méthacrylate.
Des monomères réticulants polyfonctionnels utilisables et comprenant plusieurs groupes réactifs peuvent être par exemple les silanes hydrolysables tel que l'éthyltriéthoxysilane et l'éthyltriméthoxysilane ; les silanes hydrolysables époxylés, tel que le 2-(3,4-époxycyclohexyl)éthyltriéthoxysilane et le 3-glycidoxypropyltriméthyoxysilane ; les polyisocyanates, tels que le 1,4-diisocyanatobutane, le 1,6-diisocyanatohexane, le 1,4-phénylènediisocyanate, et le 4,4'-oxybis(phénylisocyanate) ; les époxides insaturés, tel que le glycidyl méthacrylate et l'allylglycidyl éther ; les polyépoxides, tel que le diglycidyl éther, le 1,2,5,6-diépoxyhexane, et l'éthylèneglycoldiglycidyl éther.
Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel que un vinyl éther, un allyl éther, un ester acrylate ou un ester méthacrylate. De tels monomères réticulants peuvent être par exemple le diméthacrylate éthoxylé de biphénol A, le diméthacrylate éthoxylé de biphénol F, et le triméthylol propane triméthacrylate éthoxylé.
D'autres monomères réticulants éthoxylés utilisables dans la présente invention sont par exemple les agents réticulants dérivés des polyols éthoxylés divulgués dans le brevet US 6 140 435.
Des exemples particulièrement préférés de monomères réticulants sont des esters acrylates et méthacrylates de polyols ayant au moins deux groupes ester acrylate ou méthacrylate, tel que le triméthylolpropane triacrylate (TMPTA), le triméthylolpropane diméthacrylate, le triéthylène glycol diméthacrylate (TEGDMA), et le diméthacrylate de bisphénol A éthoxylé (30) (EOBDMA).
Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur variant de 0,001 à 5% en poids, de préférence de 0,05 à 2% en poids, mieux de 0,1 à 1% en poids par rapport au poids total du mélange de monomères.

Le mélange de monomères peut comprendre un ou plusieurs agents de transfert de chaîne. Les agents de transferts de chaîne sont des composés bien connus de l'état de la technique.
On peut citer en particulier les composés thiolés, les composés disulfures, tels que les C₁-C₁₈ mercaptans, les acides mercaptocarboxyliques, les esters d'acides mercaptocarboxyliques, les thioesters, les C₁-C₁₈ alkyl disulfures, les aryldisulfures, les thiols polyfonctionnels ; les phosphites et hypophosphites ; les composés haloalkyl, tel que le tétrachlorure de carbone, le bromotrichlorométhane ; et les agents de transfert de chaîne insaturés, tel que l'alphaméthylstyrène.
Les thiols polyfonctionnels sont par exemple les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérytritol-hexa-(thioglyconate).
De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.
De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.
Lorsqu'il(s) est (sont) présents, le ou les agents de transferts de chaîne sont ajoutés au mélange de monomères de préférence jusqu'à 10% en poids par rapport au poids total du mélange de monomère. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un
ou plusieurs stabilisateurs polymériques pour l'obtention de dispersions ou d'émulsions stables. De préférence, les polymères sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine; les polymères naturels modifiés, tels que la méthylcellulose, l'hydroxypropylcellulose, la carboxyméthylcellulose, les hydroxyéthylcelluloses allyliques.
Les stabilisateurs polymériques sont utilisés en une quantité au plus égale à 2 % en poids par rapport au poids total de l'émulsion, de préférence en une quantité comprise 0,0001 et 1 % en poids, mieux entre 0,01 et 0,5 % en poids par rapport au poids du mélange de monomères.

Selon un mode de réalisation préféré, le mélange de monomères comprend, par rapport au poids total du mélange de monomères :
a) de 10 à 70 % en poids du ou des monomères vinyliques substitués par un ou plusieurs groupes amino,
b) de 20 à 80 % en poids du ou des monomères vinyliques non ioniques hydrophobes,
c) de 0,001 à 25 % en poids du ou des monomères vinyliques associatifs,
d) de 0 à 25 % en poids du ou des monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs stabilisateurs polymériques.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10 % en poids du ou des agents de transfert de chaîne,
h) de 0 à 2 % en poids du ou des stabilisateurs polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisis parmi :
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6 (1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes :

   CH₂=CH-O(CH₂)ₐO(C₃H₆O)_{b}(C₂H₄O)_{c}H

   ou

   CH₂=CHCH₂O(C₃H₆O)_{d}(C₂H₄O)ₑH;

   où
   a est égal à 2, 3, ou 4 ;
   b est un entier variant de 1 à 10 ;
   c est un entier variant de 5 à 50 ;
   d est un entier variant de 1 à 10 ; et
   e est un entier variant de 5 à 50.
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs stabilisateurs polymériques.

Les polymères cationiques (i) préférés selon l'invention sont des polymères issus de la polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination CARBOPOL AQUA CC POLYMER et qui correspond à la dénomination INCI POLYACRYLATE-1 CROSSPOLYMER.
Le POLYACRYLATE-1 CROSSPOLYMER est le produit de la polymérisation d'un mélange de monomères comprenant (ou constitué de):
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1% en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.
Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyles sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (initiateur) tel que le persulfate de sodium, ou tout autre catalyseur de polymérisation par addition adaptée, comme cela est bien connu dans le domaine des polymères. La réaction est agitée jusqu'à ce que la polymérisation soit complète, généralement pendant une durée variant de 4 heures à 16 heures. L'émulsion de monomères peut être chauffée à une température comprise entre 20 et 80°C avant l'addition de l'initiateur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en solides qui varie entre 10 et 40 % en poids. Généralement, la quantité totale de polymères dans l'émulsion obtenue varie entre 15 et 35% en poids, en général au plus 25 % en poids.
Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.
Tous types de tensioactifs non ioniques, anioniques, amphotères ou cationiques classiquement utilisés dans les polymérisations en l'émulsion peuvent être utilisés.
La polymérisation peut être effectuée en présence d'un ou plusieurs initiateurs de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).

Par silicone aminée, on entend au sens de la présente invention, toute silicone comportant une ou plusieurs fonctions amine primaire, secondaire, tertiaire ou un ou plusieurs groupements ammonium quaternaire.

Les silicones aminées utilisées dans la composition cosmétique selon la présente invention sont choisies parmi
les silicones aminées ayant une ou plusieurs fonctions amines primaires de formule suivante : 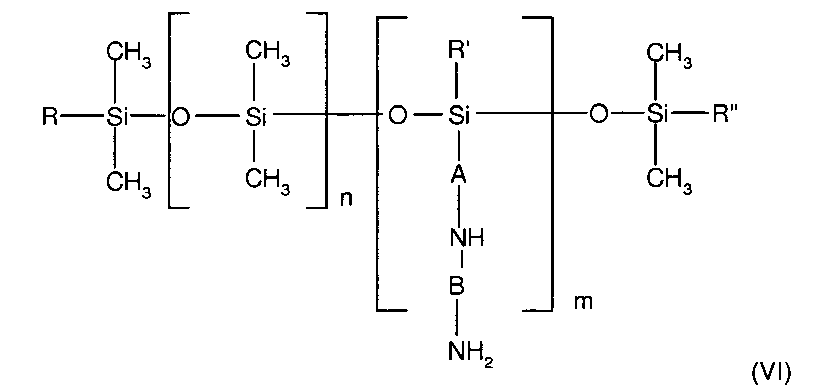 dans laquelle R, R', R", identiques ou différents, désignent un radical alkyle en C₁-C₄, de préférence CH₃ ; un radical alcoxy en C₁-C₄, de préférence méthoxy ; ou OH ; A et B représentent indépendemment un radical alkylène, linéaire ou ramifié, en C₂-C₈,
Sous réserve que R et R" ne désignent pas simultanément hydroxy,
A représente de préférence un alkylène en C₃-C₆; B représente de préférence un alkylène en C₂-C₄;
m et n sont des nombres entiers dépendant du poids moléculaire et dont la somme va de 1 à 2000.

Selon une première variante de la formule (VI), R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
Dans cette catégorie de composés, on peut citer entre autres, le produit Belsil®ADM 652, commercialisée par Wacker.
Selon une deuxième variante de la formule (VI), R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
Plus particulièrement, on peut citer le produit FluidWR® 1300, commercialisé par Wacker.

Une troisième variante de la formule (VI), est en particulier le polymère dénommé dans le dictionnaire CTFA "triméthylsilylamodiméthicone", répondant à la formule (VII) suivante : 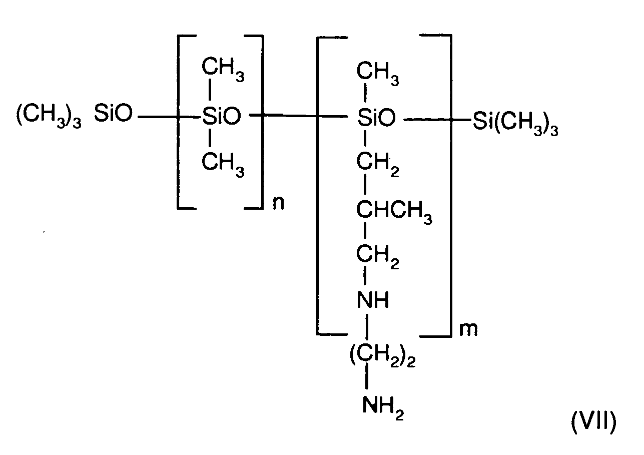 dans laquelle n et m ont les significations données ci-dessus conformément à la formule (VI).
De tels composés sont décrits par exemple dans EP 95238; un composé de formule (VII) est par exemple vendu sous la dénomination Q2-8220 par la société OSI.

Notons que la masse moléculaire de ces silicones est déterminée par chromatographie par perméation de gel (température ambiante, étalon polystyrène ; colonnes p styragem ; éluant THF ; débit de 1 mm/m ; on injecte 200 µl d'une solution à 0,5 % en poids de silicone dans le THF et l'on effectue la détection par réfractométrie et UV-métrie).

Lorsque ces composés sont mis en oeuvre, une forme de réalisation particulièrement intéressante est leur utilisation conjointe avec des agents de surface cationiques et/ou non ioniques.

Un autre produit commercial utilisable selon l'invention est le produit vendu sous la dénomination "Dow Corning Q2 7224" par la Société Dow Corning, comportant en association le triméthylsilylamodiméthicone de formule (VII) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Selon l'invention, toutes ces silicones peuvent également être utilisées sous forme d'émulsions ou de microémulsions.

Selon l'invention, la ou les silicones aminées définies ci-dessus peuvent représenter de 0,001 % à 20 % en poids, de préférence de 0,01 % à 10% en poids et plus particulièrement de 0,1 à 3% en poids par rapport au poids total de la composition finale.

Selon un mode de réalisation particulier, les compositions selon l'invention comprennent en outre une ou plusieurs silicones autres que celles de l'invention ou un autre agent bénéfique pour les matières kératiniques en particulier les cheveux tels que notamment les esters d'acides carboxyliques en C1-C30 et d'alcools mono ou polyhydroxylés en C1-C30, les huiles végétales, animales, minérales ou de synthèse, les cires, les céramides, les pseudocéramides et les polymères cationiques.

Les silicones additionnelles utilisables conformément à l'invention sont en particulier des polyorganosiloxanes insolubles dans la composition et peuvent se présenter sous forme d'huiles, de cires, de résines ou de gommes.

Ces organopolysiloxanes additionnels sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones additionnelles sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : avec 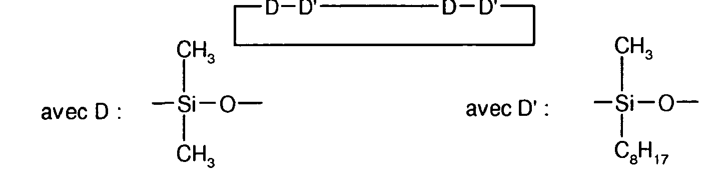 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

Ces silicones additionnelles sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les gommes de silicone utilisables en tant qu'additif sont notamment des polydiorganosiloxanes ayant des masses molaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes polydiméthylsiloxane
- les gommes polydiméthylsiloxanes/méthylvinylsiloxane,

Des produits plus particulièrement utilisables sont des mélanges tels que :
les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids molaire moyen en nombre de 500 000 solubilisée dans l'huile SF 1202 Silicone Fluid correspondant au décaméthylcyclopentasiloxane ;
les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables en tant qu'additif sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Selon l'invention, toutes les silicones peuvent être utilisées tel quel ou sous forme de solutions, d'émulsions, de nanoémulsions ou de microémulsions.

Les silicones particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 cSt, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;
- la résine d'organopolysiloxane commercialisée sous la dénomination DOW CORNING 593 ;

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un ou plusieurs agents tensioactifs qui sont généralement présent en une quantité comprise entre 0,01% et 50% en poids environ, de préférence entre 0,1% et 40% et encore plus préférentiellement entre 0,5% et 30%, par rapport au poids total de la composition.

Cet ou ces agents tensioactifs peuvent être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Ainsi, à titre d'exemple de tensioactifs anioniques utilisables, seuls ou mélanges, dans le cadre de la présente invention, on peut citer notamment (liste non limitative) les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
Parmi les alkyl (C₈-C₂₀) amidoalkyl (C₁-C₆) bétaïnes on peut citer la cocoamidopropylbétaïne vendue notamment par GOLDSCHMIDT sous le nom de TEGOBETAINE F50.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂-N(R₃)(R₄)(CH₂COO-) (X)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂-N(B)(C) (XI)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.
(iv) Les tensioactifs cationiques peuvent être choisis parmi :
   A) les sels d'ammonium quaternaires de la formule générale (XII) suivante : 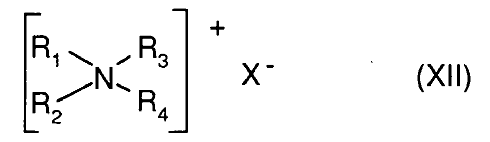 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
      , et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido
         alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (XIII) suivante : 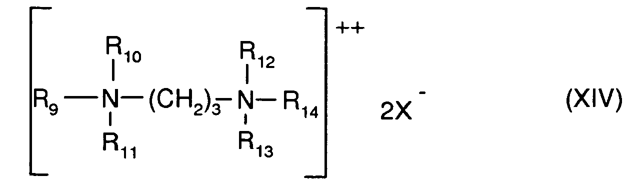
   dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (XIV) : 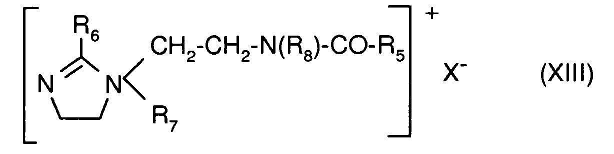 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire contenant au moins une fonction ester de formule (XV) suivante : 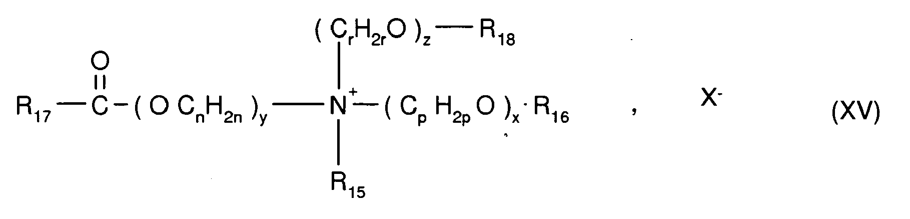
   dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :

   - le radical 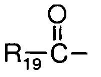
   - les radicaux R₂₀ hydrocarbonés en C₁-C₂₂ linéaires ou ramifiés, saturés ou insaturés,
   - l'atome d'hydrogène,
   - R₁₈ est choisi parmi :

   - le radical 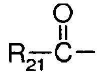
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XV) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :

- le radical 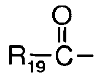
- les radicaux méthyle, éthyle ou hydrocarbonés en C₁₄₋C₂₂
- l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés
- R₁₈ est choisi parmi :

- le radical 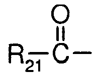
- l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et un ou plusieurs agent tensioactif amphotère.

La composition de l'invention peut également contenir un ou plusieurs additif choisi parmi les épaississants, les agents antipelliculaires ou anti-séborrhéiques, les agents anti-chute des cheveux, les parfums, les agents nacrants, les hydroxyacides, les électrolytes, les esters d'acides gras, les conservateurs, les filtres solaires siliconés ou non, les vitamines, les provitamines telles que le panthénol, les polymères anioniques ou non ioniques, les protéines, les hydrolysats de protéines, l'acide méthyl-18 eicosanoique, les huiles fluorées ou perfluorées, les amines grasses, les acides gras et leurs dérivés, les alcools gras et leurs dérivés ainsi que les mélanges de ces différents composés.
et tout autre additif classiquement utilisé dans le domaine cosmétique qui n'affecte pas les propriétés des compositions selon l'invention.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Le milieu physiologiquement et notamment cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol; les alkylèneglycols comme le propylèneglycol, les éthers de glycols.

De préférence, la composition comprend de 50 à 95 % en poids d'eau par rapport au poids total de la composition et plus particulièrement de 60 à 90% en poids.

Les compositions selon l'invention présentent un pH final généralement compris entre 3 et 10. De préférence, ce pH est compris entre 4 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine
ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage.
Dans une première variante préférée, les compositions sont des compositions lavantes et moussantes pour les cheveux et/ou la peau.

En particulier, les compositions selon l'invention sont des compositions détergentes moussantes telles que des shampooings, des gels-douche et des bains moussants, des produits démaquillants. Dans ce mode de réalisation de l'invention, les compositions comprennent au moins un ou plusieurs tensioactifs détergents.

Le ou les tensioactifs peuvent alors être indifféremment choisis, seuls ou en mélanges, au sein des tensioactifs anioniques, amphotères, non ioniques notamment tels que définis ci-dessus.
Dans les compositions conformes à l'invention sous forme de compositions détergentes en particulier les shampooings, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.
Un mélange particulièrement préféré est un mélange comprenant au moins un ou plusieurs agents tensioactifs anioniques et au moins un ou plusieurs agents tensioactifs amphotères.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société COGNIS et la cocoamidopropylbétaïne vendue notamment par GOLDSCHMIDT sous le nom de TEGOBETAINE F50.

La quantité minimale en tensioactif est alors celle juste suffisante pour conférer à la composition finale un pouvoir moussant et/ou détergent satisfaisant.

Ainsi, selon l'invention, le tensioactif détergent peut représenter de 3 % à 30 % en poids, de préférence de 6 % à 25 % en poids, et encore plus préférentiellement de 8 % à 20 % en poids, du poids total de la composition finale.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Dans une deuxième variante préférée, lorsque la composition se présente sous la forme d'un après-shampooing éventuellement à rincer, elle comprend avantageusement un ou plusieurs tensioactifs cationiques, la concentration des dits tensioactifs allant généralement de 0,1 à 10% en poids et de préférence de 0,5 à 5% en poids par rapport au poids total de la composition.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.
Les compositions selon l'invention peuvent se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée
ou sous forme de mousse. De telles formes de conditionnement sont indiquées, par exemple, lorsqu'on souhaite obtenir un spray, une laque ou une mousse pour le traitement des cheveux.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau après un éventuel temps de pause.

Ainsi, ce procédé selon l'invention permet le maintien de la coiffure, le traitement, le soin ou le lavage ou le démaquillage de la peau, des cheveux ou de toute autre matière kératinique.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a préparé un après-shampooing de composition suivante :

| | |
|---|---|
| Alcool stéarylique⁽¹⁾ | 2g |
| Polyacrylate-1 crosspolymer en émulsion à 20 % (en poids) dans l'eau ⁽²⁾ | |
| (Copolymère d'esters d'acide acrylique ou méthacrylique, de méthacrylate de C1-4 dialkylamino C1-6 alkyl, de | 0.2 gMA |
| PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate) Poly diméthyl/méthyl aminoéthyl aminopropyl siloxane en | 0.8 gMA |
| microémulsion non ionique ⁽³⁾ | |
| Chlorure de béhényl triméthyl ammonium en solution dans un mélange eau/isopropanol ⁽⁴⁾ | 1.58 gMA |
| Méthosulafte de méthyl alkyl alkylamidoéthyl imidazolinium en solution dans le propylène glycol (5) | 2.5 gMA |
| Acide lactique | Qsp pH 4 |
| Chlorhydrate de chlorhexidine | 0,03 g |
| P-hydroxybenzoate de méthyle | 0,3 g |
| Cire de candelilla | 0,4 g |
| Parfum | 0,4 g |
| Eau | Qsp 1 00g |

| | |
|---|---|
| ⁽¹⁾ vendu sous la dénomination commerciale Lanette 18 par la société Cognis ⁽²⁾ vendu sous la dénomination commerciale Carbopol Aqua CC Polymer par la société Noveon ⁽³⁾ vendu sous la dénomination SME253 par la société Momentive performance materials ⁽⁴⁾ vendu sous la dénomination Genamin KDMP par la société Clariant ⁽⁵⁾ vendu sous la dénomination Varisoft W575 PG par la société Goldschmidt | |

Cette composition est stable dans le temps et présente une texture épaisse. La composition confère du démêlage, de la souplesse, de la brillance et du lissage aux cheveux.

### EXEMPLE 2

On a préparé un après-shampooing de composition suivante :

| Alcool stéarylique⁽¹⁾ | 2 |
|---|---|
| Polyacrylate-1 crosspolymer en émulsion à 20 % (en poids) dans l'eau ⁽²⁾ | 0,2 gMA |
| Poly dimethylsiloxane à groupement aminoethyl iminopropyle en microémulsion non ionique à 17 % e(en poids) dans l'eau (3) | 0,9 gMA |
| Chlorure de béhényl triméthyl ammonium en solution dans un mélange eau/isopropanol ⁽⁴⁾ | 1,58 gMA |
| Méthosulafte de méthyl alkyl alkylamidoéthyl imidazolinium en solution dans le propylène glycol (5) | 2,5 gMA |
| Acide lactique | Qsp pH 4 |
| Chlorhydrate de chlorhexidine | 0,03 g |
| P-hydroxybenzoate de méthyle | 0,3 g |
| Cire de candelilla | 0,4 g |
| Alcool stearylique⁽¹⁾ | 2g |
| Parfum | 0,4 g |
| Eau | Qsp 100g |

| | |
|---|---|
| ⁽¹⁾ vendu sous la dénomination commerciale Lanette 18 par la société Cognis ⁽²⁾ vendu sous la dénomination commerciale Carbopol Aqua CC Polymer par la société Noveon ⁽³⁾ vendu sous la dénomination Wacker-belsil ADM LOG1 par la société Wacker ⁽⁴⁾ vendu sous la dénomination Genamin KDMP par la société Clariant ⁽⁵⁾ vendu sous la dénomination Varisoft W575 PG par la société Goldschmidt | |

Cette composition est stable dans le temps et présente une texture épaisse. La composition confère du démêlage, de la souplesse, de la brillance et du lissage aux cheveux.

### EXEMPLE 3

On a préparé une composition de shampooing :

| | |
|---|---|
| Lauryl éther sulfate de sodium (2.2 OE) en solution aqueuse (TEXAPON AOS 225 UP de COGNIS) | 12 gMA |
| Cocoyl bétaine en solution aqueuse (DEHYTON AB 30 de COGNIS) | 5 gMA |
| ACIDE LAURYL ETHER CARBOXYLIQUE (4.5 OE) (AKYPO RLM 45 CA de KAO) | 0,9 gMA |
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther | 0,6 gMA |
| de PEG 20-25 C10-30, de méthacrylate d'hydroxy C2-6 alkyl réticulé avec de l'éthylène glycol diméthacrylate en émulsion à 20% en poids dans l'eau (CARBOPOL AQUA CC POLYMER de NOVEON) Hydroxyéthyl cellulose quaternisée par chlorure de 2,3 époxypropyl triméthyl ammonium | 0,6 g |
| (JR400 AMERCHOL) Polydiméthyl siloxane à groupements aminoéthyl aminoisobutyles, à groupements terminaux triméthylsiloxy | 1 g |
| (DC 2-8566 AMINO FLUID de DOW CORNING) Conservateurs | 1,1 g |
| mica-dioxyde de titane-oxyde de fer brun | 0,005 g |
| Parfum | 0,5 |
| Oléate de propylène glycol polyéthoxylé (55 OE) et de propylène glycol en solution hydroglycolique (ANTIL 141 de GOLDSCHMIDT) | <0,2 gMA |
| Acide citrique ou hydroxyde de sodium | QS pH 5,3 |
| Eau desionisee | QS 100 g |

Cette composition présente une bonne texture stable dans le temps. Appliquée sur les cheveux, elle confère brillance et lissage.

### EXEMPLE 4 (hors invention)

On a préparé un après-shampooing de composition suivante :

| Alcool stéarylique⁽¹⁾ | 2 |
|---|---|
| Polyacrylate-1 crosspolymer en émulsion à 20 % (en poids) dans l'eau ⁽²⁾ | 0,2 gMA |
| Quaternium-80 en solution à 50% MA dans le propylène glycol (ABIL QUAT 3272 de GOLDSCHMIDT) | 0,375 gMA |
| Chlorure de béhényl triméthyl ammonium en solution dans un mélange eau/isopropanol ⁽⁴⁾ | 1,58 gMA |
| Méthosulafte de méthyl alkyl alkylamidoéthyl imidazolinium en solution dans le propylène glycol (5) | 2,5 gMA |
| Acide lactique | Qsp pH 4 |
| Chlorhydrate de chlorhexidine | 0,03 g |

| P-hydroxybenzoate de méthyle | 0,3 g |
|---|---|
| Cire de candelilla | 0,4 g |
| Alcool stearylique⁽¹⁾ | 2g |
| Parfum | 0,4 g |
| Eau | Qsp 100g |

| | |
|---|---|
| ⁽¹⁾ vendu sous la dénomination commerciale Lanette 18 par la société Cognis ⁽²⁾ vendu sous la dénomination commerciale Carbopol Aqua CC Polymer par la société Noveon ⁽⁴⁾ vendu sous la dénomination Genamin KDMP par la société Clariant ⁽⁵⁾ vendu sous la dénomination Varisoft W575 PG par la société Goldschmidt | |

Cette composition est stable dans le temps et présente une texture épaisse. La composition confère du démêlage, de la souplesse, de la brillance et du lissage aux cheveux.

## Revendications

1. Composition cosmétique, **caractérisée par le fait qu'**elle comprend, dans un milieu cosmétiquement acceptable :
(i) un ou plusieurs polymères cationiques qui sont produits par polymérisation d'un mélange de monomères comprenant :
a) un ou plusieurs monomères vinyliques substitués par un ou plusieurs groupes amino, choisis parmi :
- les mono-(C₁-C₄)alkylamino(C₁-C₈)alkyl(méth)acrylates,
- les di-(C₁-C₄)alkylamino(C₁-C₈)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides hétérocycliques comprenant un atome d'azote,
- les (méth)acrylates hétérocycliques comprenant un atome d'azote,
et leurs mélanges.
b) un ou plusieurs monomères vinyliques non ioniques hydrophobes, choisis parmi ceux de formules (I) et (II) :
(I) CH₂=C(X)Z,
(II) CH₂=CH-OC(O)R;
dans lesquelles :
X représente H ou un groupe méthyle ;
Z est choisi parmi les groupes -C(O)OR¹, -C(O)NH₂, -C(O)NHR¹, -C(O)N(R¹)₂, - C₆H₅, -C₆H₄R¹, -C₆H₄OR¹, -C₆H₄Cl, -CN, -NHC(O)CH₃, -NHC(O)H, N-(2-pyrrolidonyle), N-caprolactamyle, -C(O)NHC(CH₃)₃, -C(O)NHCH₂CH₂-NH-CH₂CH₂-urée, -SiR₃, -C(O)O(CH₂)ₓSiR₃, -C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀, un groupe alkyle en C₂-C₃₀ hydroxylé, ou un groupe alkyle en C₁-C₃₀ halogéné et
c) un ou plusieurs monomères vinyliques associatifs choisis parmi les composés de formule (III) 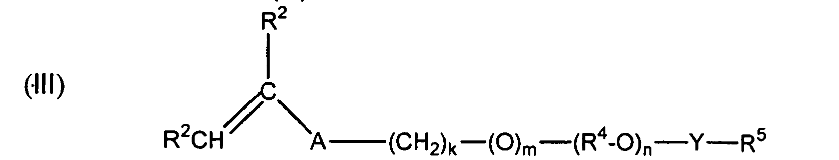 dans laquelle :
chaque R² est indépendamment H, un groupe méthyle, un groupe -C(O)OH, ou un groupe -C(O)OR³ : et R³ est un alkyle en C₁-C₃₀;
A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)- ;
Ar est un groupe aryle divalent;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno; et
e) un ou plusieurs monomères vinyliques non ioniques hydroxylés, choisis parmi les (méth)acrylates d'hydroxyalkyle en C₁-C₆, les (hydroxyalkyl en C₁-C₄)(méth)acrylamides, et leurs mélanges; et
(ii) une ou plusieurs silicones aminées choisies parmi les silicones aminées ayant une ou plusieurs fonctions amines primaires de formule suivante : 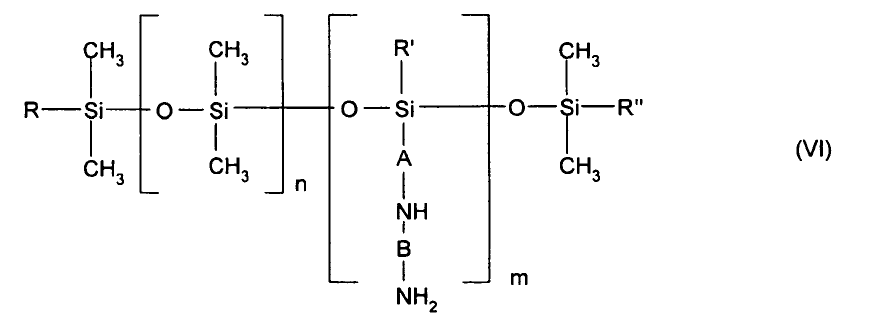 dans laquelle :
R, R', R", identiques ou différents, désignent un radical alkyle en C₁-C₄, de préférence CH₃ ; un radical alcoxy en C₁-C₄, de préférence méthoxy ; ou OH ; A et B représentent indépendamment un radical alkylène, linéaire ou ramifié, en C₂-C₈,
sous réserve que R et R" ne désignent pas simultanément hydroxy,
A représente de préférence un alkylène en C₃-C₆; B représente de préférence un alkylène en C₂-C₄;
m et n sont des nombres entiers dépendant du poids moléculaire et dont la somme va de 1 à 2000.

2. Composition selon la revendication 1, **caractérisé en ce que** le ou les monomères vinyliques substitués par un ou plusieurs groupes amino sont choisis parmi:
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle;
- les mono- ou di-(alkyl en C₁-C₄)amino(alkyl en C₁-C₄)(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

3. Composition selon l'une quelconque des revendications précédentes, **caractérisée en ce que** le ou les monomères vinyliques substitués par un ou plusieurs groupes amino représentent de 10 à 70 % en poids, de préférence de 20 à 60 % en poids, par rapport au poids total du mélange de monomères.

4. Composition selon l'une quelconque des revendications précédentes, **caractérisée en ce que** le ou les monomères vinyliques non ioniques hydrophobes sont choisis parmi les (méth)acrylates d'alkyle en C₁-C₃₀, les (alkyl en C₁-C₃₀)(méth)acrylamides, le styrène, les styrènes substitués, les esters de vinyle, les nitriles insaturés, et les silanes insaturés.

5. Composition selon l'une quelconque des revendications précédentes, **caractérisée en ce que** le ou les monomères vinyliques non ioniques hydrophobes représentent généralement de 20 à 80% en poids, de préférence de 20 à 70% en poids, par rapport au poids total du mélange de monomères.

6. Composition selon l'une quelconque des revendications précédentes, **caractérisée en ce que** le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.

7. Composition selon l'une quelconque des revendications précédentes, **caractérisée en ce que** le ou les monomères vinyliques associatifs représentent de 0,001 à 25 % en poids, de préférence de 0,01 à 15 % en poids du mélange de monomères.

8. Composition selon l'une quelconque des revendications précédentes, **caractérisée en ce que** le mélange de monomères comprend un ou plusieurs monomères tensioactifs vinyliques semi-hydrophobes d) choisis parmi les composés de formule (IV) ou (V) : 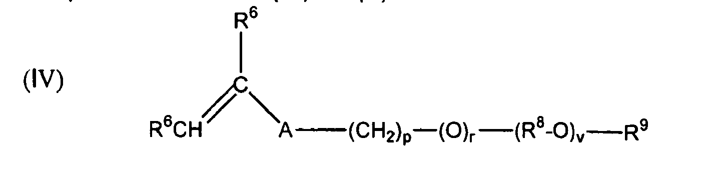
(V) D-A-(CH₂)ₚ-(O)ᵣ-(R⁸-O)ᵥ-R⁹
où, dans chaque formule (IV) ou (V),
chaque R⁶ représente indépendamment H, un alkyle en C₁-C₃₀, -C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z} -NHC(O)NH-, ou -CH₂CH₂NHC(O)- ;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et
lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄;
D est un alcényle en C₈-C₃₀ ou un alcényle en C₈-C₃₀ substitué par un groupe carboxy.

9. Composition selon la revendication 8, **caractérisée en ce que** le mélange de monomères comprend un ou plusieurs monomères tensioactifs vinyliques semi-hydrophobes ayant l'une des formules suivantes :
CH₂=CH-O(CH₂)ₐO(C₃H₆O)_{b}(C₂H₄O)_{c}H
ou
CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

10. Composition selon l'une quelconque des revendications 8 à 9, **caractérisée en ce que** le ou les monomères tensioactifs vinyliques semi-hydrophobes représentent de 0 à 25 % en poids, de préférence de 0,1 à 10 % en poids du mélange de monomères.

11. Composition selon l'une quelconque des revendications précédentes, **caractérisée en ce que** le monomère vinylique non ionique hydroxylé est le méthacrylate de 2-hydroxyéthyle.

12. Composition selon l'une quelconque des revendications précédentes, **caractérisée en ce que** le ou les monomères vinyliques non ioniques hydroxylés représentent de préférence de 0,01 à 10 % en poids du mélange de monomères.

13. Composition selon l'une quelconque des revendications précédentes, **caractérisée en ce que** le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10% en poids du ou des agents de transfert de chaîne,
h) de 0 à 2 % en poids du ou des stabilisateurs polymériques.

14. Composition selon l'une quelconque des revendications précédentes **caractérisée en ce que** ledit mélange de monomères comprend :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycollpolypropylèneglycol 30/5,
- un méthacrylate d'hydroxy(alkyle en C₂-C₆),
- un diméthacrylate d'éthylèneglycol.

15. Composition selon l'une quelconque des revendications précédentes, **caractérisée en ce que** le ou les polymères cationiques (i) sont présents à une concentration allant de 0,01 à 10% en poids par rapport au poids total de la composition, de préférence de 0,05 à 5% en poids et mieux de 0,1 à 1% en poids.

16. Composition selon l'une quelconque des revendications précédentes, **caractérisée par le fait que** la silicone aminée ii) présente la formule (VI) dans laquelle R, R', R", identiques ou différents, représentent un radical alcoxy en C₁-C₄ ou hydroxyle, l'un au moins des radicaux R ou R" est un radical alcoxy.

17. Composition selon l'une quelconque des revendications précédentes, **caractérisée par le fait que** la silicone aminée de formule (VI) est la silicone répondant à la formule (VII) suivante : 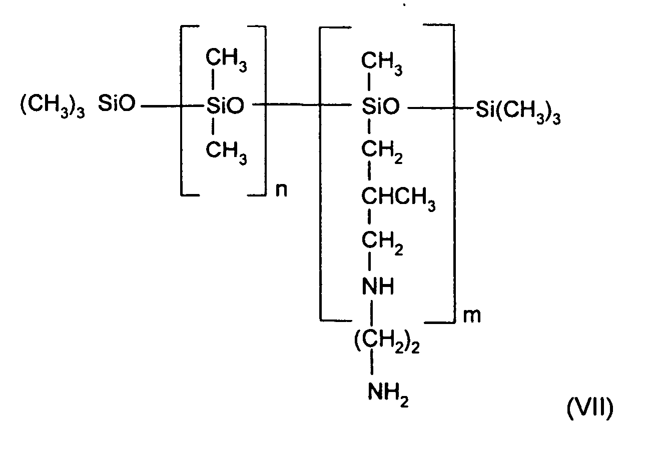 dans laquelle m et n sont des nombres entiers dépendant du poids moléculaire et dont la somme va de 1 à 2000.

18. Composition selon l'une quelconque des revendications précédentes, **caractérisée en ce que** la silicone aminée (ii) est présente à une concentration comprise entre 0,001 % et 20 % en poids par rapport au poids total de la composition, de préférence entre 0,01 % et 10 % en poids.

19. Composition selon l'une quelconque des revendications précédentes, **caractérisée par le fait qu'**elle comprend en outre une ou plusieurs silicones non aminées.

20. Composition selon la revendication 19, **caractérisée en ce que** la ou les silicones non aminées additionnelles différentes des silicones (ii) sont présentes à une concentration comprise entre 0,001 % et 20 % en poids par rapport au poids total de la composition, de préférence entre 0,01 % et 10 % en poids.

21. Composition selon l'une quelconque des revendications précédentes, **caractérisée par le fait qu'**elle comprend en outre au moins un ou plusieurs agents bénéfiques pour les matières kératiniques choisis parmi les esters d'acides carboxyliques en C1-C30 et de d'alcools mono ou polyhydroxylés en C1-C30, les huiles végétales, animales, minérales ou de synthèse, les cires, les céramides, les pseudocéramides et les polymères cationiques.

22. Utilisation d'une composition telle que définie dans l'une quelconque des revendications précédentes pour le lavage ou pour le soin des matières kératiniques.

23. Procédé de traitement des matières kératiniques, telles que les cheveux, **caractérisé en ce qu'**il consiste à appliquer sur lesdites matières une composition cosmétique selon l'une quelconque des revendications 1 à 21, puis à effectuer éventuellement un rinçage à l'eau, après un éventuel temps de pause.

24. Utilisation d'une composition telle que définie dans l'une quelconque des revendications 1 à 21 pour apporter une ou plusieurs propriétés choisies parmi la brillance, la légèreté des cheveux, la douceur, le lissage au toucher et/ou de la souplesse aux cheveux.

## Claims

1. Cosmetic composition, **characterized in that** it comprises, in a cosmetically acceptable medium:
(i)- one or more cationic polymers, which are produced by polymerization of a monomer mixture comprising:
a) one or more vinyl monomers substituted with one or more amino groups
- mono(C₁-C₄)alkylamino(C₁-C₈) alkyl (meth)acrylates,
- di(C₁-C₄)alkylamino(C₁-C₈)alkyl (meth)acrylates,
- mono(C₁-C₄)alkylamino(C₁-C₈)alkyl(meth)acrylamides,
- di(C₁-C₄) alkylamino (C₁-C₈)alkyl(meth)acrylamides,
- heterocyclic (meth)acrylamides comprising a nitrogen atom,
- heterocyclic (meth)acrylates comprising a nitrogen atom,
and mixtures thereof,
b) one or more hydrophobic nonionic vinyl monomers chosen from those of formulae (I) and (II):
(I) CH₂=C(X)Z,
(II) CH₂=CH-OC(O)R;
in which:
X represents H or a methyl group;
Z is chosen from the groups -C(O)OR¹, -C(O)NH₂, -C(O)NHR¹, -C(O)N(R¹)₂, -C₆H₅, -C₆H₄R¹, -C₆H₄OR¹, -C₆H₄Cl, - CN, -NHC(O)CH₃, -NHC(O)H, N-(2-pyrrolidonyl), N-caprolactamyl, -C(O) NHC (CH₃)₃, -C(O)NHCH₂CH₂-NH-CH₂CH₂-urea, -SiR₃, -C(O)O(CH₂)ₓSiR₃, -C(O)NH(CH₂)ₓSiR₃ and - -(CH₂)ₓSiR₃;
x represents an integer ranging from 1 to 6;
each R independently represents a C₁-C₃₀ alkyl group;
each R¹ independently represents a C₁-C₃₀ alkyl group, a C₂-C₃₀ hydroxyalkyl group or a C₁-C₃₀ haloalkyl group and
c) one or more associative vinyl monomers chosen from the compounds of formula (III): 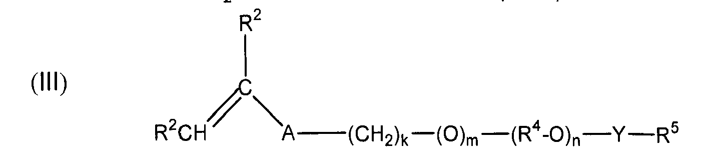 in which:
each R² is independently H, a methyl group, a group -C(O)OH or a group -C(O)OR³; and R³ is a C₁-C₃₀ alkyl;
A is a group -CH₂C(O)O-, -C(O)O-, -O-, CH₂O, -NHC(O)NH-, -C(O)NH-, -Ar-(CE₂)_{z}-NHC(O)O-, -Ar-(CE₂)_{z}-NHC(O)NH- or -CH₂CH₂-NHC(O)-;
Ar is a divalent aryl group;
E is H or a methyl group;
z is equal to 0 or 1;
k is an integer ranging from 0 to 30;
m is equal to 0 or 1, on condition that when k = 0, m = 0, and when k ranges from 1 to 30, m is equal to 1;
(R⁴-O)ₙ is a polyoxyalkylene, which is a homopolymer, a random copolymer or a block copolymer, with C₂-C₄ oxyalkylene units,
R⁴ is C₂H₄, C₃H₆, C₄H₈, or mixtures thereof,
n is an integer ranging from 5 to 250,
Y is -R⁴O-, -R⁴NH-, -C(O)-, -C(O)NH-, R⁴NHC(O)NH- or -C(O)NHC(O)-;
R⁵ is a substituted or unsubstituted alkyl chosen from linear C₈-C₄₀ alkyls, branched C₈-C₄₀ alkyls, C₈-C₄₀ alicyclics, phenyls substituted with a C₂-C₄₀ alkyl group, C₂-C₄₀ alkyls substituted with an aryl group, and C₈-C₈₀ complex esters,
the alkyl group R⁵ optionally comprising one or more substituents chosen from hydroxyl, alkoxy and halo groups; and
e) one or more hydroxylated nonionic vinyl monomers chosen from C₁-C₆ hydroxyalkyl (meth)acrylates and (C₁-C₄ hydroxyalkyl)(meth)acrylamides, and mixtures thereof,
(ii) one or more amino silicones chosen from amino silicones containing one or more primary amine functions having the following formula: 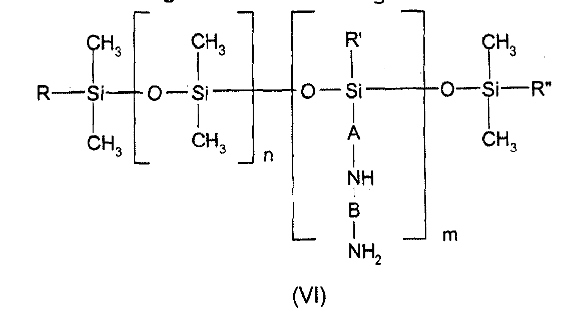 in which:
R, R' and R", which may be identical or different, denote a C₁-C₄ alkyl radical, preferably CH₃; a C₁-C₄ alkoxy radical, preferably methoxy; or OH; A and B independently represent a linear or branched C₂-C₈ alkylene radical,
with the proviso that R and R" do not simultaneously denote hydroxyl,
A preferably represents a C₃-C₆ alkylene; B preferably represents a C₂-C₄ alkylene;
m and n are integers that depend on the molecular weight and whose sum ranges from 1 to 2000.

2. Composition according to Claim 1, **characterized in that** the vinyl monomer(s) substituted with one or more amino groups is (are) chosen from:
- mono- or di(C₁-C₄ alkyl) amino (C₁-C₄ alkyl) (meth)acrylates, such as 2-(N,N-dimethylamino)ethyl (meth)acrylate, 3-(N,N-dimethylamino)propyl (meth)-acrylate, 4-(N,N-dimethylamino)butyl (meth)acrylate, (N,N-dimethylamino)-t-butyl (meth)acrylate, 2-(N,N-diethylamino)ethyl (meth)acrylate, 3-(N,N-diethylamino)propyl (meth)acrylate, 4-(N,N-diethylamino)butyl (meth)acrylate, 2-(N,N-dipropylamino)ethyl (meth)-acrylate, 3-(N,N-dipropylamino)propyl (meth)acrylate and 4-(N,N-dipropylamino)butyl (meth)acrylate;
- mono- or di(C₁-C₄ alkyl)amino(C₁-C₄alkyl)-(meth)acrylamides such as N'-(2-N,N-dimethylamino)-ethyl(meth)acrylamide and N'-(3-N,N-dimethylamino)-propylacrylamide;
- (meth)acrylamides or (meth)acrylates with a heterocyclic group comprising a nitrogen atom, such as N-(2-pyridyl)acrylamide, N-(2-imidazolyl)methacrylamide, 2-(4-morpholinyl)ethyl methacrylate, 2-(4-morpholinyl)ethyl acrylate, N-(4-morpholinyl)methacrylamide and N-(4-morpholinyl)acrylamide; and
- nitrogenous heterocycles containing vinyl group(s), such as 2-vinylpyridine and 4-vinylpyridine.

3. Composition according to any one of the preceding claims, **characterized in that** the vinyl monomer(s) substituted with one or more amino groups represent(s) from 10% to 70% by weight and preferably from 20% to 60% by weight relative to the total weight of the monomer mixture.

4. Composition according to any one of the preceding claims, **characterized in that** the hydrophobic nonionic vinyl monomer(s) is (are) chosen from C₁-C₃₀ alkyl (meth)acrylates, (C₁-C₃₀ alkyl)(meth)acrylamides, styrene, substituted styrenes, vinyl esters, unsaturated nitriles and unsaturated silanes.

5. Composition according to any one of the preceding claims, **characterized in that** the hydrophobic nonionic vinyl monomer(s) generally represent(s) from 20% to 80% by weight and preferably from 20% to 70% by weight relative to the total weight of the monomer mixture.

6. Composition according to any one of the preceding claims, **characterized in that** the associative vinyl monomer(s) is (are) chosen from polyethoxylated cetyl (meth)acrylates, polyethoxylated cetearyl (meth)acrylates, polyethoxylated stearyl (meth)acrylates, polyethoxylated arachidyl (meth)acrylates, polyethoxylated behenyl (meth)acrylates, polyethoxylated lauryl (meth)acrylates, polyethoxylated cerotyl (meth)acrylates, polyethoxylated montanyl (meth)acrylates, polyethoxylated melissyl (meth)acrylates, polyethoxylated lacceryl (meth)acrylates, polyethoxylated 2,4,6-tris(1'-phenylethyl)phenyl (meth)acrylates, polyethoxylated hydrogenated castor oil (meth)acrylates, polyethoxylated canola (meth)acrylates, polyethoxylated cholesteryl (meth)acrylates, and mixtures thereof, in which the polyethoxylated portion of the monomer comprises from 5 to 100, preferably from 10 to 80 and better still from 15 to 60 ethylene oxide units.

7. Composition according to any one of the preceding claims, **characterized in that** the associative vinyl monomer(s) represent(s) from 0.001% to 25% by weight and preferably from 0.01% to 15% by weight of the monomer mixture.

8. Composition according to any one of the preceding claims, **characterized in that** the monomer mixture comprises one or more semi-hydrophobic vinyl surfactant monomers d)
chosen from the compounds of formula (IV) or (V): 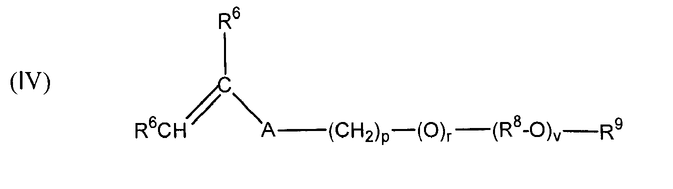
(V) D—A—(CH₂)ₚ—(O)ᵣ—(R⁸-O)ᵥ—R⁹
in which, in each formula (IV) or (V),
each R⁶ independently represents H, a C₁-C₃₀ alkyl, -C(O)OH or C(O)OR⁷;
R⁷ is a C₁-C₃₀ alkyl;
A is a group -CH₂C(O)O-, -C(O)O-, -O-, -CH₂O, -NHC(O)NH-, -C(O)NH-, -Ar- (CE₂)_{z}-NHC(O)O-, -Ar-(CE₂)_{z}-NHC(O)NH- or -CH₂CH₂NHC(O)-;
Ar is a divalent aryl group;
E is H or a methyl group;
z is equal to 0 or 1;
p is an integer ranging from 0 to 30;
r is equal to 0 or 1, on condition that when p is equal to 0, r is equal to 0, and when p ranges from 1 to 30, r is equal to 1,
(R₈-O)ᵥ is a polyoxyalkylene which is a homopolymer, a random copolymer or a block copolymer with C₂-C₄ oxyalkylene units, in which R⁸ is C₂H₄, C₃H₆, C₄H₈ or mixtures thereof, and v is an integer ranging from 5 to 250;
R⁹ is H or a C₁-C₄ alkyl;
D is a C₈-C₃₀ alkenyl or a C₈-C₃₀ alkenyl substituted with a carboxyl group.

9. Composition according to Claim 8, **characterized in that** the monomer mixture comprises one or more semi-hydrophobic vinyl surfactant monomers having one of the following formulae:
CH₂=CH-O(CH₂)ₐO(C₃H₆O)_{b}(C₂H₄O)_{c}H
or
CH₂=CHCH₂O(C₃H₆O)_{d}(C₂H₄O)ₑH;
in which:
a is equal to 2, 3 or 4;
b is an integer ranging from 1 to 10;
c is an integer ranging from 5 to 50;
d is an integer ranging from 1 to 10; and
e is an integer ranging from 5 to 50.

10. Composition according to any one of the preceding Claims 8 to 9, **characterized in that** the semi-hydrophobic vinyl surfactant monomer(s) represent(s) from 0 to 25% by weight and preferably from 0.1% to 10% by weight of the monomer mixture.

11. Composition according to any one of the preceding claims, **characterized in that** the hydroxylated nonionic vinyl monomer is 2-hydroxyethyl methacrylate.

12. Composition according to any one of the preceding claims, **characterized in that** the hydroxylated nonionic vinyl monomer(s) represent(s) preferably from 0.01% to 10% by weight of the monomer mixture.

13. Composition according to any one of the preceding claims, **characterized in that** the monomer mixture comprises, relative to the total weight of the monomer mixture:
a) from 20% to 60% by weight of vinyl monomer(s) substituted with one or more amino groups,
b) from 20% to 70% by weight of hydrophobic nonionic vinyl monomer(s),
c) from 0.01% to 15% by weight of associative vinyl monomer(s),
d) from 0.1% to 10% by weight of semi-hydrophobic vinyl surfactant monomer(s),
e) from 0.01% to 10% by weight of hydroxylated nonionic vinyl monomer(s),
f) from 0.001% to 5% by weight of crosslinking monomer(s),
g) from 0.001% to 10% by weight of chain-transfer agent(s), and
h) from 0 to 2% by weight of polymeric stabilizer(s).

14. Composition according to any one of the preceding claims, **characterized in that** the said monomer mixture comprises:
- a di(C₁-C₄alkyl)amino(C₁-C₆alkyl) methacrylate,
- one or more C₁-C₃₀ alkyl esters of (meth) acrylic acid,
- a C₁₀-C₃₀ alkyl methacrylate polyethoxylated with 20 to 30 mol of ethylene oxide,
- a 30/5 polyethylene glycol/polypropylene glycol allyl ether,
- a hydroxy(C₂-C₆ alkyl) methacrylate,
- an ethylene glycol dimethacrylate.

15. Composition according to any one of the preceding claims, **characterized in that** the cationic polymer(s) (i) is (are) present in a concentration ranging from 0.01% to 10% by weight, preferably from 0.05% to 5% by weight and better still from 0.1% to 1% by weight relative to the total weight of the composition.

16. Composition according to any one of the preceding claims, **characterized in that** the amino silicone ii) has the formula (VI) in which R, R' and R", which may be identical or different, denote a C₁-C₄ alkoxy or hydroxyl radical, and at least one of the radicals R and R" is an alkoxy radical.

17. Composition according to any one of the preceding claims, **characterized in that** the amino silicone of formula (VI) is the silicone corresponding to formula (VII) below: 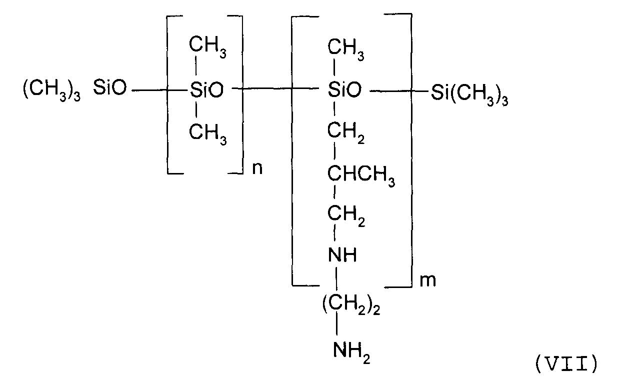 in which m and n are integers which are dependent on the molecular weight, and whose sum ranges from 1 to 2000.

18. Composition according to any one of the preceding claims, **characterized in that** the amino silicone (ii) is present in a concentration of between 0.001% and 20% by weight and preferably between 0.01% and 10% by weight relative to the total weight of the composition.

19. Composition according to any one of the preceding claims, **characterized in that** it also comprises one or more non-amino silicones.

20. Composition according to Claim 19, **characterized in that** the different additional non-amino silicones are present in a concentration of between 0.001% and 20% by weight and preferably between 0.01% and 10% by weight relative to the total weight of the composition.

21. Composition according to any one of the preceding claims, **characterized in that** it also comprises at least one or more agents that are beneficial to keratin materials, chosen from esters of C₁-C₃₀ carboxylic acids and of C₁-C₃₀ monohydroxylated or polyhydroxylated alcohols, plant, animal, mineral or synthetic oils, waxes, ceramides, pseudoceramides and cationic polymers.

22. Use of a composition as defined in any one of the preceding claims, for washing or caring for keratin materials.

23. Process for treating keratin materials, such as the hair, **characterized in that** it consists in applying to the said materials a cosmetic composition according to any one of Claims 1 to 21, optionally followed by rinsing with water, after an optional leave-in time.

24. Use of a composition as defined in any one of Claims 1 to 21, for giving the hair one or more properties chosen from sheen, lightness, softness, a smooth feel and/or suppleness.

## Patentansprüche

1. Kosmetische Zusammensetzung, **dadurch gekennzeichnet, dass** sie in einem kosmetisch unbedenklichen Medium:
(i) ein oder mehrere kationische Polymere, die durch Polymerisation einer Monomerenmischung, die
a) ein oder mehrere Vinylmonomere, die durch eine oder mehrere Aminogruppen substituiert sind, ausgewählt aus:
- Mono (C₁-C₄) alkylamino(C₁-C₈) alkyl (meth) acrylaten,
- Di (C₁-C₄) alkylamino (C₁-C₈) alkyl (meth) acrylaten,
- Mono(C₁-C₄) alkylamino (C₁-C₈)alkyl (meth) acrylamiden,
- Di (C₁-C₄) alkylamino(C₁-C₈) alkyl (meth) acrylamiden,
- heterocyclischen (Meth)acrylamiden mit einem Stickstoffatom,
- heterocyclischen (Meth)acrylaten mit einem Stickstoffatom
und Mischungen davon,
b) ein oder mehrere hydrophobe nichtionische Vinylmonomere, ausgewählt aus denjenigen der Formeln (I) und (II):
(I) CH₂=C(X)Z,
(II) CH₂=CH-OC(O)R;
worin:
X für H oder eine Methylgruppe steht;
Z aus den Gruppen -C(O)OR¹, -C(O)NH₂, -C(O)NHR¹, -C(O)N(R¹)₂, -C₆H₅, -C₆H₄R¹, -C₆H₄OR¹, -C₆H₄Cl, -CN, -NHC(O)CH₃, -NHC(O)H, N-(2-Pyrrolidonyl), N-Caprolactamyl, -C(O)NHC(CH₃)₃, -C(O) NHCH₂CH₂-NH-CH₂CH₂-Harnstoff, -SiR₃, -C(O)O(CH₂)ₓSiR₃, -C(O)NH(CH₂)ₓSiR₃ und -(CH₂)ₓSiR₃ ausgewählt ist;
x für eine ganze Zahl von 1 bis 6 steht;
R jeweils unabhängig voneinander für eine C₁-C₃₀-Alkylgruppe steht;
R¹ jeweils unabhängig voneinander für eine C₁-C₃₀-Alkylgruppe, eine C₂-C₃₀-Hydroxyalkylgruppe oder eine C₁-C₃₀-Halogenalkylgruppe steht; und
c) ein oder mehrere assoziative Vinylmonomere, ausgewählt aus den Verbindungen der Formel (III) 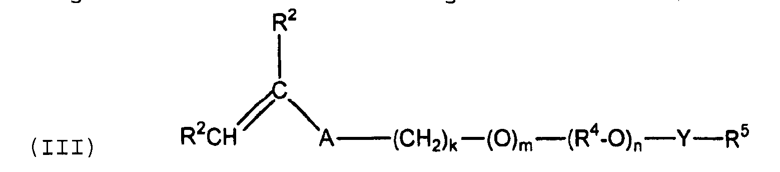 worin:
R² jeweils unabhängig voneinander H, eine Methylgruppe, eine Gruppe -C(O)OH oder eine Gruppe -C(O)OR³ ist und R³ ein C₁-C₃₀-Alkyl ist;
A eine Gruppe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z}-NHC(O)NH- oder -CH₂CH₂-NHC(O)- ist;
Ar eine zweiwertige Arylgruppe ist;
E H oder eine Methylgruppe ist;
z gleich 0 oder 1 ist;
k eine ganze Zahl im Bereich von 0 bis 30 ist;
m gleich 0 oder 1 ist, mit der Maßgabe, dass dann, wenn k = 0, m = 0, und dann, wenn k im Bereich von 1 bis 30 liegt, m gleich 1 ist;
(R⁴-O)ₙ ein Polyoxyalkylen, das ein Homopolymer, ein statistisch aufgebautes Copolymer oder ein Blockcopolymer ist, mit C₂-C₄-Oxyalkyleneinheiten ist,
R⁴ C₂H₄, C₃H₆, C₄H₈ oder Mischungen davon ist;
n eine ganze Zahl im Bereich von 5 bis 250 ist;
Y -R⁴O-, -R⁴NH-, -C(O)-, -C(O)NH-, R⁴NHC(O)NH- oder -C(O)NHC(O)- ist;
R⁵ ein gegebenenfalls substituiertes Alkyl, ausgewählt aus linearen C₈-C₄₀-Alkylgruppen, verzweigten C₈-C₄₀-Alkylgruppen, alicyclischen C₈-C₄₀-Gruppen, durch eine C₂-C₄₀-Alkylgruppe substituierten Phenylgruppen, durch eine Arylgruppe substituierten C₂-C₄₀-Alkylgruppen und C₈-C₈₀-Komplexestern, ist,
wobei die Alkylgruppe R⁵ gegebenenfalls einen oder mehrere aus Hydroxyl-, Alkoxy- und Halogengruppen ausgewählte Substituenten umfasst; und
e) ein oder mehrere hydroxylierte nichtionische Vinylmonomere, ausgewählt aus C₁-C₆-Hydroxyalkyl-(meth)acrylatan, (C₁-C₄-Hydroxyalkyl) (meth) acrylamiden und Mischungen davon
umfasst, hergestellt werden; und
(ii) ein oder mehrere Aminosilikone, ausgewählt aus Aminosilikonen mit einer oder mehreren primären Aminfunktionen der folgenden Formel: 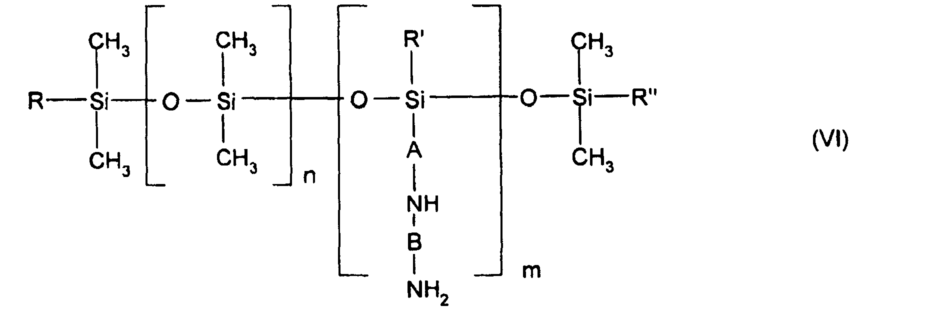 worin:
R, R' und R" gleich oder verschieden sind und für einen C₁-C₄-Alkylrest, vorzugsweise CH₃; einen C₁-C₄-Alkoxyrest, vorzugsweise Methoxy; oder OH stehen und A und B unabhängig voneinander für einen linearen oder verzweigten C₂-C₈-Alkylenrest stehen,
mit der Maßgabe, dass R und R" nicht gleichzeitig für Hydroxy stehen,
A vorzugsweise für ein C₃-C₆-Alkylen steht; B vorzugsweise für ein C₂-C₄-Alkylen steht;
m und n ganze Zahlen sind, die vom Molekulargewicht abhängen und deren Summe 1 bis 2000 beträgt;
umfasst.

2. Zusammensetzung nach Anpruch 1, **dadurch gekennzeichnet, dass** das oder die Vinylmonomere, die durch eine oder mehrere Aminogruppen substituiert sind, aus:
- Mono- oder Di(C₁-C₄-alkyl)amino(C₁-C₄-alkyl)-(meth)acrylaten, wie 2-(N,N-Dimethylamino)ethyl-(meth)acrylat, 3-(N,N-Dimethylamino)propyl(meth)-acrylat, 4-(N,N-Dimethylamino)butyl(meth)acrylat, (N,N-Dimethylamino)-t-butyl(meth)acrylat, 2-(N,N-Diethylamino)ethyl(meth)acrylat, 3-(N,N-Diethylamino)propyl(meth)acrylat, 4-(N,N-Diethylamino)-butyl(meth)acrylat, 2-(N,N-Dipropylamino)ethyl-(meth)acrylat, 3-(N,N-Dipropylamino)propyl(meth)-acrylat und 4-(N,N-Dipropylamino)butyl(meth)-acrylat;
- Mono- oder Di(C₁-C₄-alkyl)amino(C₁-C₄-alkyl)-(meth)acrylamiden, wie N'-(2-N,N-Dimethylamino)-ethyl(meth)acrylamid und N'-(3-N,N-Dimethylamino)-propylacrylamid;
- (Meth)acrylamiden oder (Meth)acrylaten mit einer ein Stickstoffatom enthaltenden heterocyclischen Gruppe, wie N-(2-Pyridyl)acrylamid, N-(2-Imidazolyl)methacrylamid, 2-(4-Morpholinyl)ethylmethacrylat, 2-(4-Morpholinyl)ethylacrylat, N-(4-Morpholinyl)methacrylamid und N-(4-Morpholinyl)-acrylamid; und
- Stickstoffheterocyclen mit mindestens einer Vinylgruppe, wie 2-Vinylpyridin und 4-Vinylpyridin;
ausgewählt sind.

3. Zusammensetzung nach einem der vorhergehenden Ansprüche, **dadurch gekennzeichnet, dass** das oder die Vinylmonomere, die durch eine oder mehrere Aminogruppen substituiert sind, 10 bis 70 Gew.-%, vorzugsweise 20 bis 60 Gew.-%, bezogen auf das Gesamtgewicht der Monomerenmischung, ausmachen.

4. Zusammensetzung nach einem der vorhergehenden Ansprüche, **dadurch gekennzeichnet, dass** das oder die hydrophoben nichtionischen Vinylmonomere aus C₁-C₃₀-Alkyl (meth) acrylaten, (C₁-C₃₀-Alkyl) (meth)-acrylamiden, Styrol, substituierten Styrolen, Vinylestern, ungesättigten Nitrilen und ungesättigten Silanen ausgewählt sind.

5. Zusammensetzung nach einem der vorhergehenden Ansprüche, **dadurch gekennzeichnet, dass** das oder die hydrophoben nichtionischen Vinylmonomere 20 bis 80 Gew.-%, vorzugsweise 20 bis 70 Gew.-%, bezogen auf das Gesamtgewicht der Monomerenmischung, ausmachen.

6. Zusammensetzung nach einem der vorhergehenden Ansprüche, **dadurch gekennzeichnet, dass** das oder die assoziativen Vinylmonomere unter polyethoxylierten Cetyl(meth)acrylaten, polyethoxylierten Cetearyl(meth)acrylaten, polyethoxylierten Stearyl(meth)acrylaten, polyethoxylierten Arachidyl(meth)acrylaten, polyethoxylierten Behenyl(meth)acrylaten, polyethoxylierten Lauryl-(meth)acrylaten, polyethoxylierten Cerotyl(meth)-acrylaten, polyethoxylierten Montanyl(meth)-acrylaten, polyethoxylierten Melissyl(meth)-acrylaten, polyethoxylierten Lacceryl(meth)-acrylaten, polyethoxylierten 2,4,6-Tri(1'-phenylethyl)phenyl(meth)acrylaten, polyethoxylierten (Meth)acrylaten von hydriertem Rizinusöl, polyethoxylierten Canola(meth)acrylaten, polyethoxylierten Cholesterin(meth)acrylaten und Mischungen davon, wobei der polyethoxylierte Teil des Monomers 5 bis 100, vorzugsweise 10 bis 80 und noch besser 15 bis 60 Ethylenoxideinheiten umfasst, ausgewählt sind.

7. Zusammensetzung nach einem der vorhergehenden Ansprüche, **dadurch gekennzeichnet, dass** das oder die assoziativen Vinylmonomere 0,001 bis 25 Gew.-%, vorzugsweise 0,01 bis 15 Gew.-% der Monomerenmischung ausmachen.

8. Zusammensetzung nach einem der vorhergehenden Ansprüche, **dadurch gekennzeichnet, dass** die Monomerenmischung ein oder mehrere semihydrophobe Vinyltensidmonomere d) umfasst, die unter den Verbindungen der Formel (IV) oder (V) ausgewählt sind: 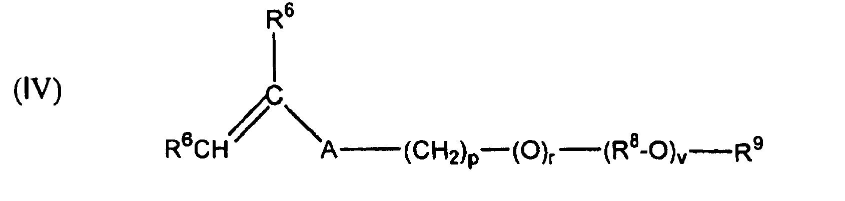
(V) D—A—(CH₂)ₚ—(O)ᵣ—(R⁸-O)ᵥ—R⁹
wobei in jeder Formel (IV) oder (V):
R⁶ jeweils unabhängig voneinander für H, ein C₁-C₃₀-Alkyl, -C(O)OH oder C(O)OR⁷ steht;
R⁷ ein C₁-C₃₀-Alkyl ist;
A eine Gruppe -CH₂C(O)O-, -C(O)O-, -O-, -CH₂O, -NHC(O)NH-, -C(O)NH-, -Ar-(CE₂)₂-NHC(O)O-, -Ar-(CE₂)_{z}-NHC(O)NH- oder -CH₂CH₂-NHC(O)- ist;
Ar eine zweiwertige Arylgruppe ist;
E H oder eine Methylgruppe ist;
z gleich 0 oder 1 ist;
p eine ganze Zahl im Bereich von 0 bis 30 ist;
r gleich 0 oder 1 ist, mit der Maßgabe, dass dann, wenn p gleich 0 ist, r gleich 0 ist, und dann, wenn p im Bereich von 1 bis 30 liegt, r gleich 1 ist;
(R₈-O)ᵥ ein Polyoxyalkylen, das ein Homopolymer, ein statistisch aufgebautes Copolymer oder ein Blockcopolymer ist, mit C₂-C₄-Oxyalkyleneinheiten ist, wobei R⁸ C₂H₄, C₃H₆, C₄H₈ oder Mischungen davon ist und v eine ganze Zahl im Bereich von 5 bis 250 ist;
R⁹ H oder ein C₁-C₄-Alkyl ist;
D ein C₈-C₃₀-Alkenyl oder ein durch eine Carboxygruppe substituiertes C₈-C₃₀-Alkenyl ist.

9. Zusammensetzung nach Anspruch 8, **dadurch gekennzeichnet, dass** die Monomerenmischung ein oder mehrere semihydrophobe Vinyltensidmonomere mit einer der folgenden Formeln umfasst:
CH₂=CH-O(CH₂)ₐO (C₃H₆O)_{b}(C₂H₄O)_{c}H
oder
CH₂=CHCH₂O (C₃H₆O)_{d} (C₂H₄O) eH;
wobei
a gleich 2, 3 oder 4 ist;
b eine ganze Zahl im Bereich von 1 bis 10 ist;
c eine ganze Zahl im Bereich von 5 bis 50 ist;
d eine ganze Zahl im Bereich von 1 bis 10 ist; und
e eine ganze Zahl im Bereich von 5 bis 50 ist.

10. Zusammensetzung nach einem der Ansprüche 8 bis 9, **dadurch gekennzeichnet, dass** das oder die semihydrophoben Vinyltensidmonomere 0 bis 25 Gew.-%, vorzugsweise 0,1 bis 10 Gew.-% der Monomerenmischung ausmachen.

11. Zusammensetzung nach einem der vorhergehenden Ansprüche, **dadurch gekennzeichnet, dass** es sich bei dem hydroxylierten nichtionischen Vinylmonomer um 2-Hydroxyethylmethacrylat handelt.

12. Zusammensetzung nach einem der vorhergehenden Ansprüche, **dadurch gekennzeichnet, dass** das oder die hydroxylierten nichtionischen Vinylmonomere vorzugsweise 0,01 bis 10 Gew.-% der Monomerenmischung ausmachen.

13. Zusammensetzung nach einem der vorhergehenden Ansprüche, **dadurch gekennzeichnet, dass** die Monomerenmischung, bezogen auf das Gesamtgewicht der Monomerenmischung, Folgendes umfasst:
a) 20 bis 60 Gew.-% eines oder mehrerer Vinylmonomere, die durch eine oder mehrere Aminogruppen substituiert sind,
b) 20 bis 70 Gew.-% eines oder mehrerer hydrophober nichtionischer Vinylmonomere,
c) 0,01 bis 15 Gew.-% eines oder mehrerer assoziativer Vinylmonomere,
d) 0,1 bis 10 Gew.-% eines oder mehrerer semihydrophober Vinyltensidmonomere,
e) 0,01 bis 10 Gew.-% eines oder mehrerer hydroxylierter nichtionischer Vinylmonomere,
f) 0,001 bis 5 Gew.-% eines oder mehrerer Vernetzungsmonomere und
g) 0,001 bis 10 Gew.-% eines oder mehrerer Kettenübertragungsmittel,
h) 0 bis 2 Gew.-% eines oder mehrerer polymerer Stabilisatoren.

14. Zusammensetzung nach einem der vorhergehenden Ansprüche, **dadurch gekennzeichnet, dass** die Monomerenmischung Folgendes umfasst:
- ein Di (C₁-C₄)alkylamino(C₁-C₆)alkyl(meth)acrylat,
- einen oder mehrere (Meth)acrylsäure-C₁-C₃₀-alkylester,
- ein mit 20 bis 30 mol Ethylenoxid polyethoxyliertes C₁₀-C₃₀-Alkylmethacrylat,
- einen Polyethylenglykol/Polypropylenglykol-(30/5)-allylether,
- ein Hydroxy(C₂-C₆-alkyl)methacrylat,
- ein Ethylenglykoldimethacrylat.

15. Zusammensetzung nach einem der vorhergehenden Ansprüche, **dadurch gekennzeichnet, dass** das oder die kationischen Polymere (i) in einer Konzentration im Bereich von 0,01 bis 10 Gew.-%, bezogen auf das Gesamtgewicht der Zusammensetzung, vorzugsweise 0,05 bis 5 Gew.-% und noch besser 0,1 bis 1 Gew.-% vorliegen.

16. Zusammensetzung nach einem der vorhergehenden Ansprüche, **dadurch gekennzeichnet, dass** das Aminosilikon ii) die Formel (VI) aufweist, worin R, R' und R'' gleich oder verschieden sind und für einen C₁-C₄-Alkoxyrest oder Hydroxyl stehen und mindestens einer der Reste R oder R'' ein Alkoxyrest ist.

17. Zusammensetzung nach einem der vorhergehenden Ansprüche, **dadurch gekennzeichnet, dass** es sich bei dem Aminosilikon der Formel (VI) um das Silikon der folgenden Formel (VII) handelt: 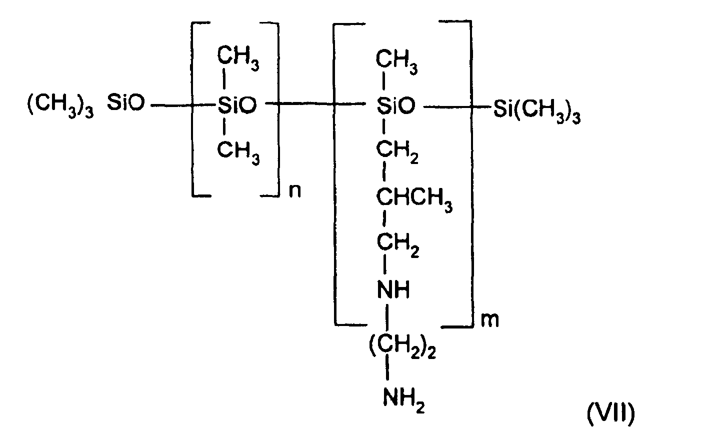 worin m und n ganze Zahlen sind, die vom Molekulargewicht abhängen und deren Summe 1 bis 2000 beträgt.

18. Zusammensetzung nach einem der vorhergehenden Ansprüche, **dadurch gekennzeichnet, dass** das Aminosilikon ii) in einer Konzentration zwischen 0,001 und 20 Gew.-%, bezogen auf das Gesamtgewicht der Zusammensetzung, vorzugsweise zwischen 0,01 und 10 Gew.-%, vorliegt.

19. Zusammensetzung nach einem der vorhergehenden Ansprüche, **dadurch gekennzeichnet, dass** sie außerdem ein oder mehrere Nichtaminosilikone umfasst.

20. Zusammensetzung nach Anspruch 19, **dadurch gekennzeichnet, dass** das oder die zusätzlichen Nichtaminosilikone, die von den Silikonen (ii) verschieden sind, in einer Konzentration zwischen 0,001 und 20 Gew.-%, bezogen auf das Gesamtgewicht der Zusammensetzung, vorzugsweise zwischen 0,01 und 10 Gew.-%, vorliegen.

21. Zusammensetzung nach einem der vorhergehenden Ansprüche, **dadurch gekennzeichnet, dass** sie außerdem mindestens ein oder mehrere für Keratinmaterialien vorteilhafte Agentien umfasst, die aus Estern von C₁-C₃₀-Carbonsäuren und mono- oder polyhydroxylierten C₁-C₃₀-Alkoholen, pflanzlichen, tierischen, mineralischen oder synthetischen Ölen, Wachsen, Ceramiden, Pseudoceramiden und kationischen Polymeren ausgewählt sind.

22. Verwendung einer Zusammensetzung gemäß einem der vorhergehenden Ansprüche zum Waschen oder Pflegen von Keratinmaterialien.

23. Verfahren zur Behandlung von Keratinmaterialien, wie den Haaren, **dadurch gekennzeichnet, dass** es darin besteht, dass man auf die Materialien eine kosmetische Zusammensetzung nach einem der Ansprüche 1 bis 21 aufbringt und danach gegebenenfalls eine Spülung durchführt, gegebenenfalls nach einer Wartezeit.

24. Verwendung einer Zusammensetzung gemäß einem der Ansprüche 1 bis 21, um den Haaren eine oder mehrere aus Glanz, Leichtigkeit, Weichheit, glattem Griff und/oder Geschmeidigkeit ausgewählte Eigenschaften zu verleihen.
